# EUROPEAN PATENT APPLICATION

(11) **EP 4 442 265 A1**
(43) Date of publication of application: **09.10.2024**
(21) Application number: 22900323.1
(22) Date of filing: 22.11.2022
(51) Int. Cl.: A61K 35/745, A61P 19/00, A23L 33/135

(54) **USE OF BIFIDOBACTERIUM LACTIS BL-99 IN PROTECTION OF CARTILAGE**

(30) Priority: 30.11.2021 CN 202111453416
(71) Applicant: Inner Mongolia Yili Industrial Group Co., Ltd., Inner Mongolia 010000 (CN); Inner Mongolia Dairy Tech Res Institute Co Ltd, Hohhot, Inner Mongolia 010110 (CN)
(72) Inventor: HUNG, Wei-Lian, Hohhot, Inner Mongolia 010000 (CN); SHI, Yujie, Hohhot, Inner Mongolia 010000 (CN); LIU, Wei-Hsien, Hohhot, Inner Mongolia 010000 (CN); FENG, Haotian, Hohhot, Inner Mongolia 010000 (CN); LIU, Biao, Hohhot, Inner Mongolia 010000 (CN); LI, Wei, Hohhot, Inner Mongolia 010000 (CN); CHEN, Qingshan, Hohhot, Inner Mongolia 010000 (CN); ZHOU, Mingqiao, Hohhot, Inner Mongolia 010000 (CN)
(74) Representative: Dehns
(86) International application number: PCT/CN2022/133511
(87) International publication number: WO 2023/098518

(57) **Abstract**

Disclosed is the use of *Bifidobacterium lactis* BL-99 in the protection of cartilage. Provided in the present invention is the use of *Bifidobacterium lactis* in the preparation of a composition for protecting cartilage, wherein the *Bifidobacterium lactis* is *Bifidobacterium lactis* with the deposit number of CGMCC No. 15650. The *Bifidobacterium lactis* can promote the development of cartilage, increase cartilage density, protect cartilage from damage, promote cartilage repair and/or ameliorate cartilage damage, and is beneficial to cartilage health.

## Description

### Technical Field

The present invention relates to *Bifidobacterium lactis* having a cartilage protection effect, and in particular, the present invention relates to the use of *Bifidobacterium lactis* BL-99 (having the deposit number of CGMCC No. 15650) in the preparation of a composition for use in the protection of cartilage and/or promotion of cartilage health.

### Background Art

Cartilage tissue is widely present in vertebrates and is an indispensable tissue type for organisms to maintain normal activities. As an important tissue type in vertebrates, cartilage participates in the occurrence and development of skeletal systems. The development process of chondrocytes is regulated by a complex network of signaling molecules. Abnormalities in key genes cause chondrocyte formation disorders and are associated with the occurrence of many congenital and acquired cartilage calcification bone diseases, such as craniofacial deformity and osteoarthritis, affecting the health of about 10-12% of the global population.

Chondroitin sulfate is an effective ingredient that is considered to have a cartilage protection effect, and can be used as a nutritional supplement. However, studies have reported that chondroitin sulfate may cause some side effects, common side effects including nausea and stomach pain, and in some cases chondroitin sulfate may also cause rash, regurgitation and alopecia.

Therefore, it is very important to develop preventive and therapeutic products with a cartilage protection efficacy without toxic and side effects.

### Summary of the Invention

An objective of the present invention is to provide the new use of *Bifidobacterium lactis.*

According to a specific embodiment of the present invention, the present invention provides the new use of a *Bifidobacterium lactis* BL-99 strain, which was deposited in the China General Microbiological Culture Collection Center on December 18, 2017 having the deposit number of CGMCC No. 15650. The *Bifidobacterium lactis* BL-99 strain is a biological material that has been disclosed in the patent document CN 110964653 A. Studies in the prior art show that the *Bifidobacterium lactis* BL-99 is a safe probiotic strain for consumption and has gastric acid resistance and intestinal juice resistance. In addition, the *Bifidobacterium lactis* BL-99 has the effects of improving the intestinal immunity, improving the intestinal barrier integrity, inhibiting the generation of intestinal inflammatory factors, etc.

By means of zebrafish model experiments, the inventors of the present invention have found in studies that *Bifidobacterium lactis* having the deposit number of CGMCC No. 15650 has a cartilage protection efficacy, which may be specifically manifested in promoting the proliferation of chondrocytes, promoting metabolic balance of chondrocytes and/or inhibiting apoptosis of chondrocytes, thereby promoting the development of cartilage, increasing cartilage density and/or ameliorating cartilage damage.

Zebrafish are very similar to humans in terms of bone development, the bone development of the zebrafish is substantially consistent with human bone biomineralization, there is an endochondral ossification process, and the fully developed zebrafish bones have a hierarchical structure consistent with human long bones. In addition, zebrafish are also very similar to humans in terms of the molecular mechanism of bone growth and development. Using transgenic zebrafish with cartilage expressed as green fluorescence, the fluorescence intensity of cartilage can be directly observed, photographed and quantitatively analyzed by a microscope.

Therefore, the present invention provides the use of *Bifidobacterium lactis* in the preparation of a composition for use in the protection of cartilage, wherein the *Bifidobacterium lactis* is *Bifidobacterium lactis* having the deposit number of CGMCC No. 15650 (i.e., the *Bifidobacterium lactis* BL-99 of the present invention).

According to a specific embodiment of the present invention, in the use of the *Bifidobacterium lactis* BL-99 of the present invention, the protection of cartilage comprises promoting the development of cartilage, increasing cartilage density, protecting cartilage from damage, promoting cartilage repair and/or ameliorating cartilage damage.

According to a specific embodiment of the present invention, in the use of the *Bifidobacterium lactis* BL-99 of the present invention, the protection of cartilage comprises promoting the proliferation of chondrocytes, promoting metabolic balance of chondrocytes and/or inhibiting apoptosis of chondrocytes.

According to a specific embodiment of the present invention, in the use of the *Bifidobacterium lactis* BL-99 of the present invention, the composition comprises a food composition, a feed composition or a pharmaceutical composition.

According to a specific embodiment of the present invention, in the use of the *Bifidobacterium lactis* BL-99 of the present invention, the *Bifidobacterium lactis* can be used in the form of a solid or liquid bacterial preparation for the preparation of the composition.

According to a specific embodiment of the present invention, in the use of the *Bifidobacterium lactis* BL-99 of the present invention, the *Bifidobacterium lactis* can be used in the form of live bacteria or inactivated bacteria for the preparation of the composition.

According to a specific embodiment of the present invention, in the use of the *Bifidobacterium lactis* BL-99 of the present invention, the *Bifidobacterium lactis* is combined with one or more of prebiotics and other probiotics to prepare a composition for use in the protection of cartilage. Preferably, the prebiotics comprise one or more of isomerized lactose, fructo-oligosaccharides, and galacto-oligosaccharides. The prebiotics may comprise other raw materials containing oligosaccharides such as inulin. Preferably, the other probiotics comprise *Bifidobacterium lactis* such as HN019. HN019 is a mature commercial strain in the field of dairy fermentation.

According to some specific embodiments of the present invention, in the use of the *Bifidobacterium lactis* BL-99 of the present invention, the composition is a pharmaceutical composition. The pharmaceutical composition further comprises a pharmaceutically acceptable auxiliary material, and the auxiliary material comprises an excipient, a diluent, a filler and/or an absorption enhancer.

According to some specific embodiments of the present invention, in the use of the *Bifidobacterium lactis* BL-99 of the present invention, the composition is a food composition. The food may be a fermented dairy product, a cheese, a dairy-containing beverage, a solid beverage or a dairy powder.

According to a specific embodiment of the present invention, the composition of the present invention may further comprise conventional material components in the art. For example, the pharmaceutical composition may comprise an appropriate amount of auxiliary material, and the auxiliary material may be an excipient, a diluent, a filler and/or an absorption enhancer. The food composition containing the *Bifidobacterium lactis* of the present invention can be produced in accordance with the food containing *Bifidobacterium lactis* in the prior art. Depending on the needs of the recipient, the composition may be in various forms, such as a powder, a lozenge, a granulate, a microcapsule and/or a liquid preparation.

According to a specific embodiment of the present invention, in the use of the *Bifidobacterium lactis* BL-99 of the present invention, the *Bifidobacterium lactis* is used in an amount of 1.0 × 10³ CFU/day-1.0 × 10¹² CFU/day, preferably 1.0 × 10⁷ CFU/day-1.0 × 10¹¹ CFU/day. When the BL-99 of the present invention is used in combination with other probiotics, the total amount of probiotics should be 1.0 × 10³ CFU/day-1.0 × 10¹² CFU/day, preferably 1.0 × 10' CFU/day-1.0 × 10¹¹ CFU/day.

When the BL-99 of the present invention is used in combination with a prebiotic, the prebiotic is used in an amount of 0.3 g/day to 30 g/day.

In another aspect, the present invention further provides a composition having a cartilage protection efficacy, wherein the composition comprises one or more of the following combinations:
*Bifidobacterium lactis* BL-99 and a prebiotic;
*Bifidobacterium lactis* BL-99 and *Bifidobacterium lactis* HN019;
*Bifidobacterium lactis* BL-99, *Bifidobacterium lactis* HN019 and a prebiotic;
preferably, the prebiotic comprises one or more of isomerized lactose, fructo-oligosaccharides, and galacto-oligosaccharides.

According to a specific embodiment of the present invention, when the composition having the cartilage protection efficacy of the present invention comprises *Bifidobacterium lactis* BL-99 and a prebiotic, the ratio of the amount of the *Bifidobacterium lactis* having the deposit number of CGMCC No. 15650 to the amount of the prebiotic is 1.0 × 10³ CFU-1.0 × 10¹² CFU: 0.3 g-30 g, preferably 1.0 × 10⁷ CFU-1.0 × 10¹¹ CFU: 0.3 g-30 g.

According to a specific embodiment of the present invention, when the composition having the cartilage protection efficacy of the present invention comprises *Bifidobacterium lactis* BL-99 and *Bifidobacterium lactis* HN019, the two bacteria can be combined in any ratio. Generally, the two bacteria are combined in a ratio of (0.01 to 100) : 1.

In another aspect, the present invention further provides a method for protecting cartilage, which method comprises administering to a subject an effective amount of *Bifidobacterium lactis* having the deposit number of CGMCC No. 15650.

According to a specific embodiment of the present invention, in the method for protecting cartilage of the present invention, the protecting cartilage comprises:
promoting the development of cartilage, increasing cartilage density, protecting cartilage from damage, promoting cartilage repair and/or ameliorating cartilage damage; or
promoting the proliferation of chondrocytes, promoting metabolic balance of chondrocytes and/or inhibiting apoptosis of chondrocytes.

According to a specific embodiment of the present invention, in the method for protecting cartilage of the present invention, the *Bifidobacterium lactis* is administered to a subject in need thereof in the form of the aforementioned composition (food composition, feed composition or pharmaceutical composition).

The present invention provides the new use of *Bifidobacterium lactis* BL-99 in the preparation of a composition for use in the protection of cartilage. By means of experiments, the present invention proves that the *Bifidobacterium lactis* BL-99 can promote the proliferation of chondrocytes, promote metabolic balance of chondrocytes and/or inhibit apoptosis of chondrocytes, thereby promoting the development of cartilage, increasing cartilage density and/or ameliorating cartilage damage.

### Brief Description of the Drawings

FIG. 1 shows typical pictures of the fluorescence intensity of zebrafish cartilage after treatment with probiotics. Note: Green fluorescence in the pictures represents zebrafish cartilage.
FIG. 2 shows fluorescence intensity detection results of zebrafish cartilage after treatment with probiotics *Bifidobacterium lactis* BL-99 and commercial strain X. *p < 0.05, **p < 0.01, ***p < 0.001, vs. model control group.

### Detailed Description of Embodiments

In order to understand the technical features, objectives and beneficial effects of the present invention more clearly, the technical solutions of the present invention are described in detail as below, but they cannot be construed as a limitation for the implementable scope of the present invention.

### Example 1

### 1. Experimental samples and materials

### 1.1. Samples

*Bifidobacterium lactis* BL-99 and *Bifidobacterium lactis* HN019 were both prepared into 2.00 mg/mL stock solutions with standard dilution water just before needed.

The solid preparation of probiotic *Bifidobacterium lactis* BL-99 was provided by Inner Mongolia Yili Industrial Group Co., Ltd..

The *Bifidobacterium lactis* HN019 was a commercial strain.

A strain control group was also set up using a commercial probiotic strain X of *Bifidobacterium lactis.*

**Positive control:** Chondroitin sulfate A sodium salt, white powder, Batch No. I2003201, Shanghai Aladdin Bio-Chem Technology Co., Ltd, stored at 4°C in the dark. A 100 mg/mL stock solution was prepared with ultrapure water just before needed.

### 1.2. Experimental animals

Zebrafish were raised in fish farming water at 28°C (water quality: 200 mg of instant sea salt was added to every 1 L of reverse osmosis water, the conductivity was 450 to 550 µS/cm; pH was 6.5 to 8.5; the hardness was 50-100 mg/L CaCO₃), with the number of the using of laboratory animal: SYXK (Zhe) 2012-0171. The raising and management complied with the requirements of international AAALAC certification (certification number: 001458).

Transgenic zebrafish with cartilage expressed as green fluorescence were bred by natural pair-wise mating. Zebrafish aged 2 days post fertilization (2 dpf) were used to determine the maximum tolerated concentration (MTC) for the cartilage protection efficacy of probiotics and to evaluate the efficacy.

### 1.3. Dose translation from zebrafish to human

The formula of equivalent dose translation from zebrafish to mammals was determined by mathematical modeling and fitting according to the publicly published (SCI entry) data of chemical drugs, natural drugs, Chinese patent medicines, and health care food. At present, the formula of equivalent dose translation from zebrafish to human used by our company was: zebrafish (mg/L) = [human (g/day) × 1000]/6. For example, the single-administration concentration for zebrafish was 1000 µg/mL, which was translated into a dose for human according to the above-mentioned formula, i.e., 6 g per day. The formula of equivalent dose translation from zebrafish to human (zebrafish (mg/L) = [human (g/day) × 1000]/6) was also applied for the health care food, and the application basis and scope were described as follows: using the health-based guidance value calculation formula (HBGV=NOAEL/UFs) and the definition of the uncertainty factors (UFs) mentioned by WHO in "Principles and Methods for the Risk Assessment of Chemicals in Food", verified by relevant literature and internal experimental data of Hunter Biotech, the uncertainty factor for the general population was 10, and the uncertainty factor for the sensitive population was 100.

### 1.4. Instruments, consumables and reagents

Dissecting microscope (SZX7, OLYMPUS, Japan); CCD camera (VertA1, Shanghai Tusen Vision Technology Co., Ltd., China); motorized multizoom fluorescence microscope (AZ100, Nikon, Japan); precision electronic balance (CP214, OHAUS, USA); 6-well plate (Nest Biotech, China).

Dexamethasone acetate (Batch No. B1828095, Shanghai Aladdin Bio-Chem Technology Co., Ltd., China), dimethyl sulfoxide (DMSO, Batch No. BCCD7238, Sigma, Switzerland); methylcellulose (Batch No. 079K0054V, Sigma, USA).

### 2. Detection method

### 2.1. Experimental principle

Dexamethasone is an artificially synthetic glucocorticoid. Dexamethasone induces zebrafish, which can disrupt the metabolic balance of zebrafish chondrocytes, inhibit cell proliferation, and cause death or apoptosis of chondrocytes, thereby causing cartilage damage. Early use of dexamethasone can relieve joint pain and inflammation. However, long-term use of dexamethasone can disrupt the metabolic balance of chondrocytes and cause death or apoptosis of chondrocytes, thereby reducing the number of chondrocytes and further aggravating the deterioration of cartilage tissue in the knee joint.

### 2.2 MTC determination

2 dpf transgenic zebrafish with cartilage expressed as green fluorescence were randomly selected and placed in a 6-well plate, with 30 zebrafish in each well (experimental group). Except for the normal control group, the remaining experimental groups were given dexamethasone dissolved in water to establish zebrafish cartilage damage models. After 24 h of treatment at 28°C, dexamethasone was removed, and *Bifidobacterium lactis* BL-99 and commercial strain X (concentrations as shown in Table 1) dissolved in water were administered, respectively, while a model control group and a normal control group were set up, with a volume of 3 mL per well. Except for the normal control group, the remaining experimental groups were again given dexamethasone dissolved in water, and each experimental group was treated with dexamethasone for another 48 h, during which time liquid exchange was performed every day. After the treatment was completed, the MTCs of the *Bifidobacterium lactis* BL-99 and the commercial strain X in model zebrafish were determined.

### 2.3. Evaluation of cartilage protection efficacy

2 dpf transgenic zebrafish with cartilage expressed as green fluorescence were randomly selected and placed in a 6-well plate, with 30 zebrafish in each well (experimental group). Except for the normal control group, the remaining experimental groups were given dexamethasone dissolved in water to establish zebrafish cartilage damage models. After 24 h of treatment at 28°C, dexamethasone was removed, and *Bifidobacterium lactis* BL-99 and commercial strain X (concentrations as shown in Table 2) dissolved in water were administered, respectively, while a model control group and a normal control group were set up, with a volume of 3 mL per well. Except for the normal control group, the remaining experimental groups were again given dexamethasone dissolved in water, and each experimental group was treated with dexamethasone for another 48 h, during which time liquid exchange was performed every day. After the treatment was completed, 10 zebrafish were randomly selected from each experimental group and photographed by a fluorescence microscope, NIS-Elements D 3.20 advanced image processing software was used to analyze and collect data, the fluorescence intensity of cartilage was analyzed, and the statistical analysis results of this indicator were used to evaluate the cartilage protection efficacy of the *Bifidobacterium lactis* BL-99 and the commercial strain X. Statistical treatment results were expressed as mean ± SE. Statistical analysis was performed with SPSS26.0 software, and p < 0.05 indicated that the difference was statistically significant.

### 3. Detection results

### 3.1. MTC

Under the experimental conditions, the MTCs of the *Bifidobacterium lactis* BL-99 and the commercial strain X in model zebrafish were 500 µg/mL, 500 µg/mL, 1000 µg/mL and 2000 µg/mL, respectively. See Table 1 for details.

**Table 1. Experimental results on the concentration for cartilage protection efficacy of probiotics (n = 30)**

| Group | Concentrati on µg/mL | Count CFU/mL | Deaths (zebrafish) | Mortality (%) | Phenotype |
|---|---|---|---|---|---|
| Normal control group | / | / | 0 | 0 | No obvious abnormality |
| Model control group | / | / | 0 | 0 | No obvious abnormality |
| *Bifidobacteriu m lactis* BL-99 | 125 | 1.26*10⁸ | 0 | 0 | Status similar to that in the model control group |
| | 250 | 2.34*10⁸ | 0 | 0 | Status similar to that in the model control group |
| | 500 | 4.98*10⁸ | 0 | 0 | Status similar to that in the model control group |
| | 1000 | 1.01*10⁹ | 0 | 0 | Status poorer than that in the model control group |
| | 2000 | Out of detection range | - | - | - |
| Commercial strain X | 125 | 0.180*10⁸ | 0 | 0 | Status similar to that in the model control group |
| | 250 | 0.420*10⁸ | 0 | 0 | Status similar to that in the model control group |
| | 500 | 0.600*10⁸ | 0 | 0 | Status similar to that in the model control group |
| | 1000 | 1.20*10⁸ | 0 | 0 | Status similar to that in the model control group |
| | 2000 | 2.61*10⁸ | 30 | 100 | - |

### 3.2. Evaluation of cartilage protection efficacy

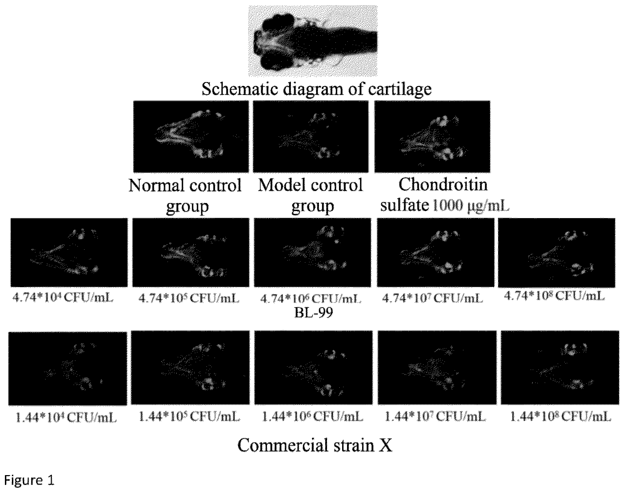

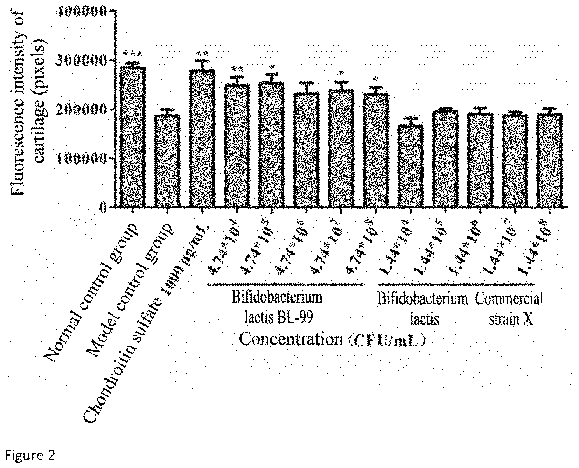

Under the experimental conditions, the *Bifidobacterium lactis* BL-99 had a cartilage protection efficacy, while the *Bifidobacterium lactis* HN019 had no significant cartilage protection efficacy. See Table 2, FIG. 1 and FIG. 2 for details.

**Table 2. Experimental results on the evaluation of cartilage protection efficacy of probiotics (n = 10)**

| Group | Concentration CFU/mL | Amount for human per day (g) | Amount for human per day (CFU/g) | Fluorescence intensity of cartilage (pixel, mean ± SE) |
|---|---|---|---|---|
| Normal control group | - | - | - | 283826 ± 9652*** |
| Model control group | | - | - | 186061 ± 12621 |
| Chondroitin sulfate | 1000 µg/mL | - | - | 277530 ± 20981** |
| *Bifidobacteriu m lactis* BL-99 | 4.74*10⁴ | 0.0003 | 4.50*10⁷ | 248008 ± 17302** |
| | 4.74*10⁵ | 0.003 | 4.50*10⁸ | 252162 ± 19399* |
| | 4.74*10⁶ | 0.030 | 4.50*10⁹ | 230991 ± 21685 |
| | 4.74*10⁷ | 0.300 | 4.50*10¹⁰ | 236812 ± 17724* |
| | 4.74*10⁸ | 3.00 | 4.50*10¹¹ | 229537 ± 14435* |
| Commercial strain X | 1.44*10⁴ | 0.0006 | 1.80*10⁷ | 164897 ± 15904 |
| | 1.44*10⁵ | 0.006 | 1.80*10⁸ | 195132 ± 5720 |
| | 1.44*10⁶ | 0.060 | 1.80*10⁹ | 189841 ± 12593 |
| | 1.44*10⁷ | 0.600 | 1.80*10¹⁰ | 187068 ± 7443 |
| | 1.44*10⁸ | 6.00 | 1.80*10¹¹ | 188542 ± 12270 |

| | | | | |
|---|---|---|---|---|
| *p < 0.05, **p < 0.01, ***p < 0.001, vs. model control group. | | | | |

The *Bifidobacterium lactis* BL-99 (intervention concentration is about 10⁸ CFU/day, corresponding to the dose for human) has the efficacies of promoting the development of cartilage, increasing cartilage density and protecting cartilage.

### Example 2. Cartilage protection efficacy of composition containing Bifidobacterium lactis BL-99 and prebiotics or other probiotics

In this example, the *Bifidobacterium lactis* BL-99 was combined with various prebiotics or other probiotics to investigate the cartilage protection efficacy of the composition. The experimental method for evaluating the cartilage protection efficacy was the same as that in Example 1.

The cartilage protection efficacy evaluation results of the compositions formed by combining the *Bifidobacterium lactis* BL-99 with various prebiotics or other probiotics in this example are as shown in Table 3.

**Table 3. Experimental results on the evaluation of cartilage protection efficacy of compositions containing Bifidobacterium lactis BL-99 and prebiotics or other probiotics (n = 10)**

| Group and amount | Amount for human per day | Fluorescence intensity of cartilage (pixel, mean ± SE) | Increased cartilage protection efficacy (%) compared with the model group |
|---|---|---|---|
| Normal control group | - | 331506 ± 20833*** | - |
| Model control group | - | 205834 ± 15373 | - |
| Sulfuric acid cartilage 1000 µg/mL | - | 296517 ± 25160** | 44 |
| *Bifidobacterium lactis* BL-99 (4.74*10⁵ CFU/mL) | 4.50*10⁸ CFU | 265318 ± 19296* | 29 |
| *Bifidobacterium lactis* BL-99 (4.74*10⁵ CFU/mL) + Isomerized lactose 62.5 µg/mL | 4.50*10⁸ CFU + 0.375 g | 303568 ± 27956** | 47 |
| *Bifidobacterium lactis* BL-99 (4.74*10⁵ CFU/mL)+ Isomerized lactose 250 µg/mL | 4.50*10⁸ CFU + 1.50 g | 329001 ± 13573*** | 60 |
| *Bifidobacterium lactis* BL-99 (4.74*10⁵ CFU/mL)+ Fructo-oligosaccharide 62.5 µg/mL | 4.50*10⁸ CFU + 0.375 g | 288975 ± 11468*** | 40 |
| *Bifidobacterium lactis* BL-99 (4.74*10⁵ CFU/mL)+ Fructo-oligosaccharide 250 µg/mL | 4.50*10⁸ CFU + 1.50 g | 313361 ± 17804*** | 52 |
| *Bifidobacterium lactis* BL-99 (4.74*10⁵ CFU/mL)+ Galacto-oligosaccharide 62.5 µg/mL | 4.50*10⁸ CFU + 0.375 g | 255114 ± 17120* | 28 |
| *Bifidobacterium lactis* BL-99 (4.74*10⁵ CFU/mL)+ Galacto-oligosaccharide 250 µg/mL | 4.50*10⁸ CFU + 1.50 g | 311695 ± 21099*** | 57 |
| *Bifidobacterium lactis* BL-99+ *Bifidobacterium lactis* HN019 (Total bacteria 4.74*10⁵CFU/mL) | 4.50*10⁸ CFU | 306386 ± 27734** | 54 |

| | | | |
|---|---|---|---|
| *p < 0.05, **p < 0.01, ***p < 0.001, vs. model control group. | | | |

It can be seen that the compositions formed by combining the *Bifidobacterium lactis* BL-99 and various prebiotics or other probiotics all have good efficacies of promoting the development of cartilage, increasing cartilage density and protecting cartilage.

## Claims

1. Use of *Bifidobacterium lactis* in the preparation of a composition for use in protection of cartilage, wherein the *Bifidobacterium lactis* is *Bifidobacterium lactis* having the deposit number of CGMCC No. 15650.

2. The use according to claim 1, wherein the protection of cartilage comprises promoting the development of cartilage, increasing cartilage density, protecting cartilage from damage, promoting cartilage repair, and/or ameliorating cartilage damage.

3. The use according to claim 1, wherein the protection of cartilage comprises promoting proliferation of chondrocytes, promoting the metabolic balance of chondrocytes, and/or inhibiting apoptosis of chondrocytes.

4. The use according to claim 1, wherein the composition comprises a food composition, a feed composition or a pharmaceutical composition.

5. The use according to claim 1, wherein the *Bifidobacterium lactis* is used in the form of a solid or liquid bacterial preparation for the preparation of the composition; and/or
the *Bifidobacterium lactis* is used in the form of live bacteria or inactivated bacteria for the preparation of the composition.

6. The use according to claim 1, wherein the *Bifidobacterium lactis* is combined with one or more of prebiotics and other probiotics to prepare a composition for use in protection of cartilage; preferably, the prebiotics comprise one or more of isomerized lactose, fructo-oligosaccharides and galacto-oligosaccharides;
preferably, the other probiotics comprise *Bifidobacterium lactis* such as HN019.

7. The use according to any one of claims 4-6, wherein the composition is a food composition.

8. The use according to claim 7, wherein the food is a fermented dairy product, cheese, a dairy beverage, a solid beverage, or milk powder.

9. The use according to any one of claims 4-8, wherein the *Bifidobacterium lactis* is taken in an amount of 1.0 × 10³ CFU/day to 1.0 × 10¹² CFU/day, preferably 1.0 × 10⁷ CFU/day to 1.0 × 10¹¹ CFU/day;
when the *Bifidobacterium lactis* is taken in combination with a prebiotic, the prebiotic is taken in an amount of 0.3 g/day to 30 g/day.

10. A composition having cartilage protection efficacy, comprising one or more of the following combinations:
*Bifidobacterium lactis* having the deposit number of CGMCC No. 15650 and a prebiotic;
*Bifidobacterium lactis* having the deposit number of CGMCC No. 15650 and *Bifidobacterium lactis* HN019;
*Bifidobacterium lactis* having the deposit number of CGMCC No. 15650, *Bifidobacterium lactis* HN019, and a prebiotic;
preferably, the prebiotic comprises one or more of isomerized lactose, fructo-oligosaccharides, and galacto-oligosaccharides.

11. The composition according to claim 10, wherein :
when the composition comprises *Bifidobacterium lactis* having the deposit number of CGMCC No. 15650 and a prebiotic, the ratio of the amount of the *Bifidobacterium lactis* having the deposit number of CGMCC No. 15650 to the amount of the prebiotic is 1.0 × 10³ CFU to 1.0 × 10¹² CFU: 0.3 g to 30 g, preferably 1.0 × 10⁷ CFU to 1.0 × 10¹¹ CFU: 0.3 g to 30 g, and/or
when the composition comprises *Bifidobacterium lactis* having the deposit number of CGMCC No. 15650 and *Bifidobacterium lactis* HN019, the two bacteria are combined in any ratio, for example, the two bacteria are combined in a ratio of (0.01 to 100) : 1.

12. A method for protecting cartilage, comprising administering to a subject an effective amount of *Bifidobacterium lactis* having the deposit number of CGMCC No. 15650.

13. The method according to claim 12, wherein the protecting cartilage comprises:
promoting the development of cartilage, increasing cartilage density, protecting cartilage from damage, promoting cartilage repair, and/or ameliorating cartilage damage; or
promoting proliferation of chondrocytes, promoting the metabolic balance of chondrocytes, and/or inhibiting apoptosis of chondrocytes.

14. The method according to claim 12 or 13, wherein the *Bifidobacterium lactis* is administered to a subject in need thereof in the form of the composition according to claim 10 or 11.
